# EUROPEAN PATENT SPECIFICATION

(11) **EP 3 910 316 B1**
(45) Date of publication and mention of the grant of the patent: **17.12.2025**
(21) Application number: 20739163.2
(22) Date of filing: 02.01.2020
(51) Int. Cl.: G01N 1/22, G01N 15/06, G01N 15/02, C12Q 1/24, B01D 49/00, G01N 21/76

(54) **KIT FOR MEASURING BIOAEROSOL AND PARTICULATE MATTER**
KIT ZUR MESSUNG VON BIOAEROSOL UND FEINSTAUB
KIT DE MESURE DE BIOAÉROSOL ET DE MATIÈRE PARTICULAIRE

(30) Priority: 08.01.2019 KR 20190002308
(43) Date of publication of application: 17.11.2021
(73) Proprietor: Kyungdong E&S Co., Ltd, Daegu (KR)
(72) Inventor: BYEON, Jeong Hoon, Gyeongsan-si, Gyeongsangbuk-do 38541 (KR)
(74) Representative: RGTH
(86) International application number: PCT/KR2020/000015
(87) International publication number: WO 2020/145565

(56) References cited:
- WO-A1-2015/130000
- KR-A- 20120 086 384
- KR-A- 20170 035 770
- KR-A- 20180 078 832
- KR-A- 20180 080 762
- KR-B1- 101 798 601
- KR-B1- 101 857 887
- KR-B1- 101 857 887
- PARK JI-WOON ET AL: "Fast Monitoring of Indoor Bioaerosol Concentrations with ATP Bioluminescence Assay Using an Electrostatic Rod-Type Sampler", vol. 10, no. 5, 7 May 2015 (2015-05-07), pages e0125251, XP055958602, Retrieved from the Internet <URL:https://journals.plos.org/plosone/article/file?id=10.1371/journal.pone.0125251&type=printable> [retrieved on 20220907], DOI: 10.1371/journal.pone.0125251

## Description

### [Technical Field]

The present disclosure relates to a kit for measuring the concentrations of an airborne bioaerosol and particulate matter.

### [Background Art]

In general, a method of measuring microbes floating in the air includes a method of measuring a bioaerosol. The method of measuring a bioaerosol includes forcibly inhaling a certain amount of the air, passing the air through a medium, and culturing microbes adsorbed onto the medium, and has an advantage in that it is possible to obtain results fairly close to the number of suspended microbes, but has drawbacks in that the results may vary according to the culture conditions or the measurement location and it takes a lot of time to obtain results.

Among a variety of conventional techniques for collecting microbes, Korean Patent No. 10-0549222 discloses the technique for collecting microbes in which the microbes in particles floating in the air are easily collected by inertial impaction by spraying the air onto a collection plate which rotates by means of a nozzle. However, the related art has drawbacks in that the microbes should be collected on the collection plate and transferred to culture equipment, and it is inconvenient to carry and move equipment due to an increase in volume thereof because a drive unit for rotating the collection plate, and the like are included.

KR 101 857 887 B1 discloses a measuring kit for a gaseous floating virus to rapidly measure concentration of a floating microorganism in the air. Provided is a measuring kit for rapid measurement, which can shorten sampling time of a virus, a germ, and mold of which a floating size range is different in the air, and selectively perform sampling.

### [Disclosure]

### [Technical Problem]

Therefore, it is an object of the present invention to provide a kit for measuring an airborne bioaerosol and particulate matter, which is capable of shortening a sampling time of germs (bacteria, and the like) and fungi (mold, and the like) which have different airborne size ranges, allowing selective sampling, and rapidly outputting a relative proportion of bacterial and fungal particles per mass of particulate matter through calculation.

### [Technical Solution]

According to the present invention, there is provided a kit for measuring an airborne bioaerosol and particulate matter according to independent claim 1. Preferred embodiments are defined in the dependent claims.

According to the invention, the kit includes a humidification unit provided to supply an aqueous solution containing a cell lysing agent toward the air flowing into the main body. Also, each of the separation unit and the swab may be replaced and installed when the airborne bioaerosols falling within different size ranges are separately collected using the sampler.

In addition, the separation unit may include a first separation unit configured to supply the airborne bioaerosol having a size range of 2.5 µm or less in the air through the first inlet port and a second separation unit configured to supply the airborne bioaerosol having a size range of 10 µm or less in the air through the first inlet port, and at least one of the first and second separation units may be optionally installed.

The airborne bioaerosol falling within the first size range may have a size range of 2.5 µm or less in the air, and the airborne bioaerosol falling within the second size range may have a size range of 10 µm or less in the air.

Additionally, the particulate matter measurement unit may measure each of fine particulate matter (PM 10) and ultrafine particulate matter (PM 2.5) to output a mass concentration value of the particulate matter per unit air volume.

Furthermore, the ATP measurement unit may further include an ATP monitor configured to output each of the measured ATP values as a relative luminescence unit (RLU) value per unit air volume.

Also, the kit may further include a user terminal capable of outputting a user interface, the control unit may be provided to receive each of the first and second data to generate third data, and the control unit may provide the generated third data to the user terminal.

In addition, the user terminal may be provided to input user input values, and the user input values may include at least one selected from a volume flow rate of sampling air, a sampling time, a colony forming unit (CFU) conversion formula, a reference value for the airborne bioaerosol according to the measurement unit for each place, and reference values for fine particulate matter and ultrafine particulate matter for each place.

Additionally, the control unit may generate a plurality of pieces of third data based on at least one of the first data, the second data, and the user input values.

Furthermore, the user interface may further include a user input window configured to input at least one of the user input values, a third data output window configured to output each of the pieces of the generated third data, and a third data schematization output window configured to provide each of the pieces of the third data in a schematized form.

Also, the third data may include at least one selected from an RLU value (RLU/µg [PMI0]) of the airborne bioaerosol having a size range of 10 µm or less in the air per unit mass of the particulate matter, an RLU value (RLU/µg [PM2.5]) of the airborne bioaerosol having a size range of 2.5 µm or less in the air per unit mass of the particulate matter, an RLU value (RLU/µg [PM10-PM2.5]) of the airborne bioaerosol having a size range of 2.5 µm to 10 µmin the air per unit mass of the particulate matter, a colony forming unit (CFU) value (CFU/µg [PMI0]) of the airborne bioaerosol having a size range of 10 µm or less per unit mass of the particulate matter, a colony forming unit (CFU) value (CFU/µg [PM2.5]) of the airborne bioaerosol having a size range of 2.5 µm or less in the air per unit mass of the particulate matter, a colony forming unit (CFU) value (CFU/µg [PM10-PM2.5]) of the airborne bioaerosol having a size range of 2.5 µm to 10 µm in the air per unit mass of the particulate matter, a colony forming unit (CFU) value (CFU/m³[PM10]) of the airborne bioaerosol having a size range of 10 µm or less in the air per unit air volume, a colony forming unit (CFU) value (CFU/m³[PM2.5]) of the airborne bioaerosol having a size range of 2.5 µm or less in the air per unit air volume, a colony forming unit (CFU) value (CFU/m³[PM10-PM2.5]) of the airborne bioaerosol having a size range of 2.5 µm to 10 µm in the air per unit air volume, and a mass concentration value (µg/m³) of the particulate matter having a particle size of greater than 2.5 µm and less than 10 µm per unit air volume. Further, the third data schematization output window may display, in the form of a horizontal straight line, each of reference lines representing a reference value for the airborne bioaerosol and/or reference values for fine particulate matter and ultrafine particulate matter at each measurement place based on the user input values.

There is provided a method of measuring an airborne bioaerosol and particulate matter using the above-described kit for measuring an airborne bioaerosol and particulate matter, which includes a bioaerosol concentration measurement step of measuring ATP in the airborne bioaerosol attached to a swab; a particulate matter concentration measurement step of measuring a concentration of the particulate matter; and a data generation step of receiving first data output from the ATP measurement unit and second data output from the particulate matter measurement unit to generate third data in the control unit. There is also provided a method of measuring an airborne bioaerosol and particulate matter using the above-described kit for measuring an airborne bioaerosol and particulate matter, which includes a bioaerosol concentration measurement step of measuring ATP in the airborne bioaerosol attached to a swab; a particulate matter concentration measurement step of measuring a concentration of the particulate matter; and a data generation step of receiving first data output from the ATP measurement unit and second data output from the particulate matter measurement unit to generate third data in the control unit.

There is also provided a method of measuring an airborne bioaerosol and particulate matter using the above-described kit for measuring an airborne bioaerosol and particulate matter, which includes a bioaerosol concentration measurement step of measuring ATP in the airborne bioaerosol attached to each of the swabs; a particulate matter concentration measurement step of measuring a concentration of the particulate matter; and a data generation step of receiving first data output from the ATP measurement unit and second data output from the particulate matter measurement unit to generate third data in the control unit.

### [ Advantageous Effects]

A kit for measuring an airborne bioaerosol and particulate matter according to one exemplary embodiment of the present invention has advantages in that the kit can shorten a sampling time of germs (bacteria, and the like) and fungi (mold, and the like) having different airborne size ranges, allow selective sampling, and rapidly output a relative proportion of bacterial and fungal particles per mass of the particulate matter or per unit air volume through calculation.

Also, the kit according to the present invention can provide real-time information on the fungi and germs included in fine particulate matter and ultrafine particulate matter floating in the air in various formats, and thus can provide a bioaerosol monitoring platform to swiftly deal with biological threats caused by the bioaerosol.

Further, the kit according to the present invention can provide a user interface capable of realizing functions, such as selecting and adjusting output information, in order to easily analyze the fine particulate matter and bioaerosol by place by a user.

### [Description of Drawings]

FIGS. I and 2 are perspective views of a measurement kit having a sampler installed therein according to a first embodiment of the present invention.
FIGS. 3 and 4 are schematic diagrams showing the sampler according to the first embodiment of the present invention.
FIGS. 5 and 6 are schematic diagrams showing a separation unit according to one embodiment of the present invention.
FIGS. 7 to 10 are diagrams for describing a control unit according to one embodiment of the present invention.
FIG. 11 is a schematic diagram showing a sampler according to a second embodiment of the present invention.
FIG. 12 is a schematic diagram showing a sampler according to a third embodiment of the present invention.

### [Best Mode]

Hereinafter, preferred embodiments of the present invention will be described in detail with reference to the accompanying drawings. Prior to the description, it should be understood that the terminology used in the specification and the appended claims should not be construed as limited to general and dictionary meanings, but interpreted based on the meanings and concepts corresponding to technical aspects of the present invention on the basis of the principle that the present inventors can appropriately define the concepts of terms for the purpose of describing the present invention in the best way.

Also, regardless of the reference numerals in the drawings, like or corresponding elements have the same or similar reference numerals, and thus a redundant description thereof is omitted for clarity. In this case, the dimensions and shapes of elements shown in the drawings may be exaggerated or diminished for the sake of convenience of description.

The present invention relates to a kit 10 capable of measuring a concentration of a bioaerosol and particulate matter in the air (hereinafter referred to as a "measurement kit").

The term "airborne bioaerosol," "suspended microbe," and "aerial bioaerosol" used in the present disclosure may refer to bacteria, viruses, germs, molds, and the like, which float in the air.

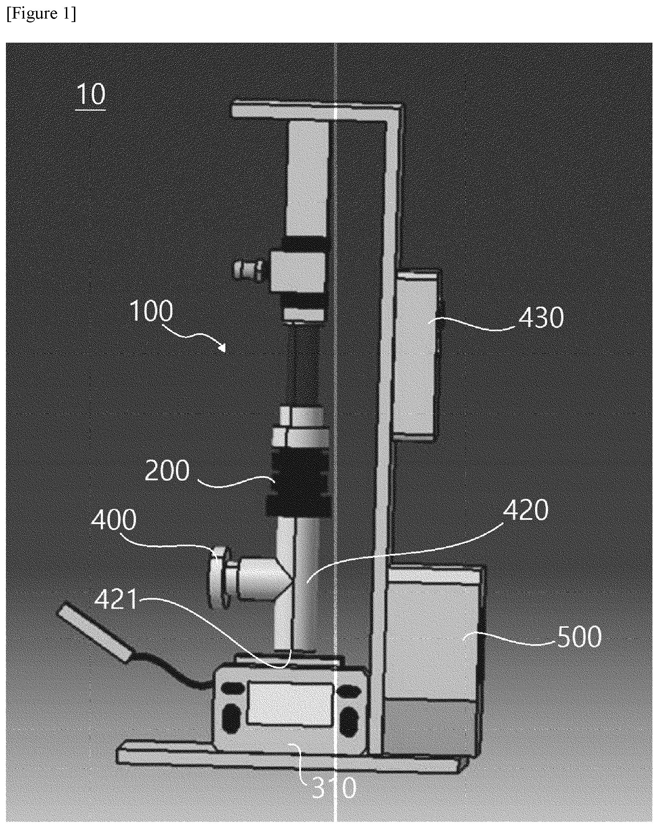

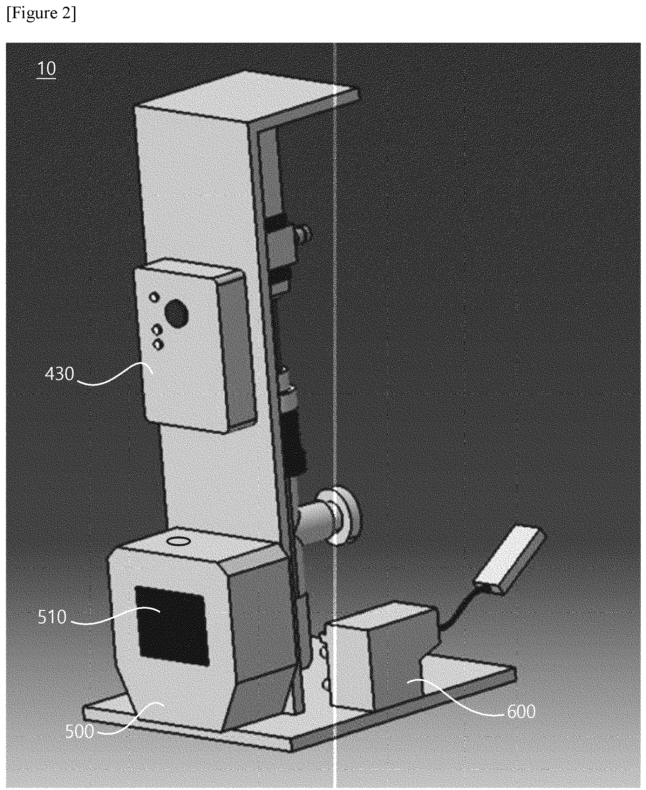

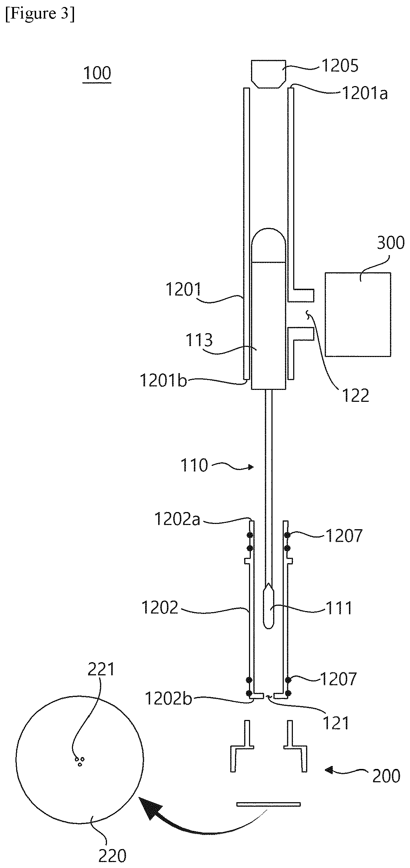

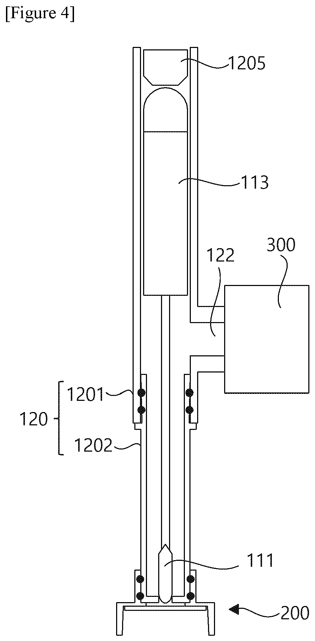

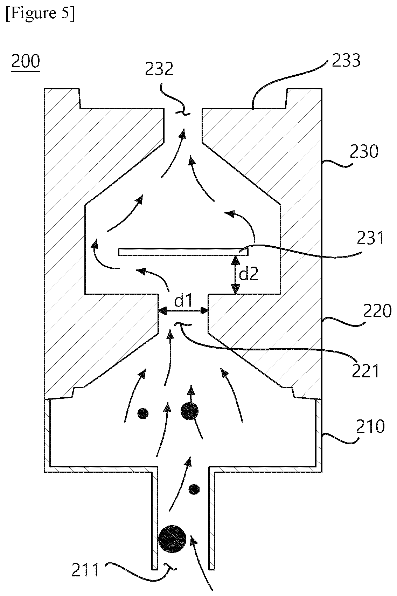

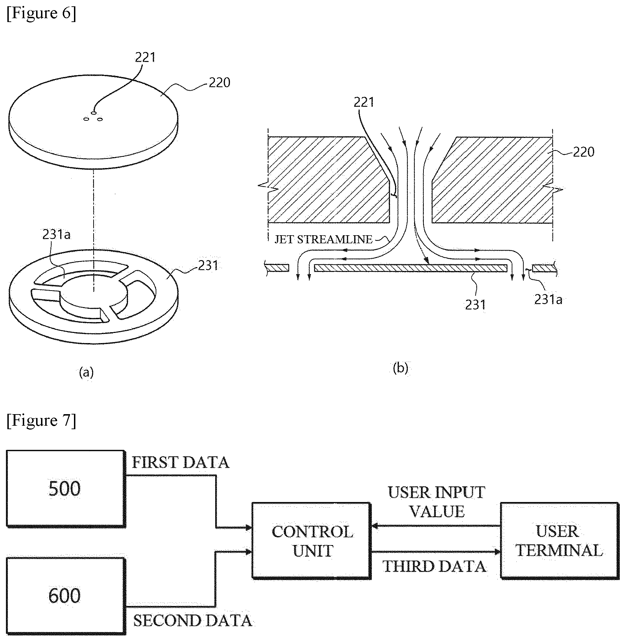

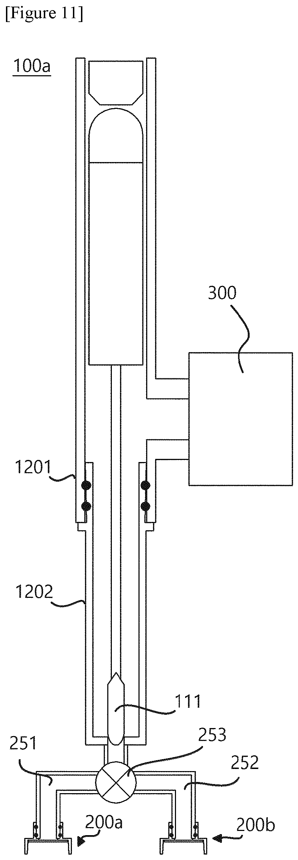

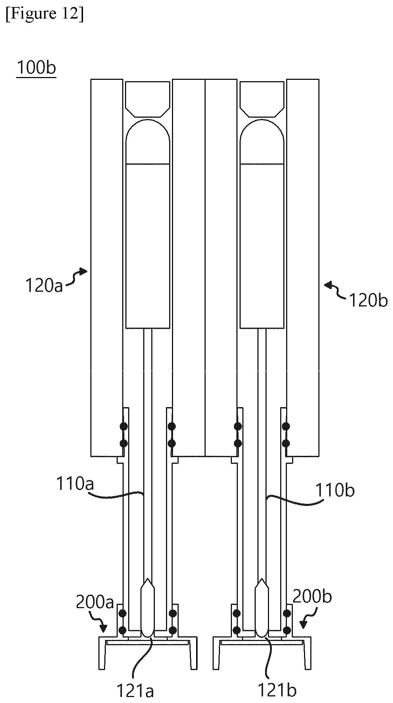

FIGS. 1 and 2 are perspective views of a measurement kit having a sampler installed therein according to a first embodiment of the present invention, FIGS. 3 and 4 are schematic diagrams showing the sampler according to the first embodiment of the present invention, FIGS. 5 and 6 are schematic diagrams showing a separation unit according to one embodiment of the present invention, FIGS. 7 to 10 are diagrams for describing a control unit according to one embodiment of the present invention, FIG. 11 is a schematic diagram showing a sampler according to a second embodiment of the present invention, and FIG. 12 is a schematic diagram showing a sampler according to a third embodiment of the present invention.

Hereinafter, a measurement kit 10 according to one embodiment of the present invention will be described in detail with reference to FIGS. 1 to 12.

Referring to FIGS. 1 and 2, the measurement kit 10 capable of measuring a concentration of an airborne bioaerosol and particulate matter according to one embodiment of the present includes a sampler 100, 100a or 100b configured to collect an airborne bioaerosol, an ATP measurement unit 500 configured to measure a concentration of the collected airborne bioaerosol, a particulate matter measurement unit 600 configured to measure a concentration of the particulate matter, and a control unit provided to receive first data output from the ATP measurement unit and second data output from the particulate matter measurement unit to generate third data.

The measurement kit 10 according to the present invention has advantages in that it is able to shorten a sampling time of germs (bacteria, and the like) and fungi (mold, and the like) having different size ranges, which float in the outside air, allow selective sampling, and rapidly output a relative proportion of bacterial and fungal particles per mass of particulate matter or per unit air volume by calculating concentration values measured through the ATP measurement unit 500 and the particulate matter measurement unit 600 by means of the control unit.

More specifically, referring to FIGS. 3 and 4, the sampler 100 according to the first embodiment of the present invention includes at least one swab 110 provided to attach the airborne bioaerosol thereto.

Also, the sampler 100 according to the first embodiment of the present invention includes a main body 120 having an internal space, wherein the swab 110 is detachably installed in the internal space, and having a first inlet port 121 and a first outlet port 122 provided, respectively, to allow outside air to be introduced and discharged therethrough.

The swab has a collection unit 111 to which the airborne bioaerosol is attached, and a fixing unit 113 extending from the collection unit 111 to fix the collection unit 111 in the internal space.

The main body 120 includes an upper holder 1201 having the first outlet port 122 provided therein, and a lower holder 1202 having the first inlet port 121 provided therein.

The upper holder 1201 and the lower holder 1202 are detachably connected so that a portion of an upper end of the lower holder 1202 is inserted into a lower end of the upper holder 1201. Therefore, the upper holder 1201 and the lower holder 1202 may be detachably coupled to each other.

Here, because a sealing member 1207 (e.g., an O-ring) is provided at a portion of the upper end of the lower holder 1202, a space between the upper holder 1201 and the lower holder 1202 may be sealed more tightly.

The first inlet port 121 may be provided at a roughly central region of the lower end 1202b of the lower holder 1202, and the collection unit 111 of the swab may be disposed adjacent to the first inlet port 121.

The fixing unit 113 of the swab is press-fitted into a portion of the upper holder 1201, and thus the collection unit 111 may be fixed so that the collection unit 111 can be disposed adjacent to the first inlet port 121.

The first outlet port 122 may be provided in a side portion of the upper holder 1201, and the fixing unit 113 of the swab may be disposed between the first outlet port 122 and an upper end 1201a of the upper holder.

Also, a capping member 1205 may be provided at the upper end 1201a of the upper holder to cap an upper end of the upper end 1201a. One example of the capping member may be a set screw, but the present invention is not limited thereto.

Here, because each of an upper end 1202a of the lower holder 1202 and a lower end 1201b of the upper holder 1201 is open so that the upper holder 1201 is fluidically connected to the lower holder 1202, the air flowing into the first inlet port 121 may flow toward the first outlet port 122.

In addition, the sampler 100 according to the first embodiment of the present invention includes at least one separation unit 200 detachably installed on the first inlet port 121 side of the main body 120 and provided to supply the airborne bioaerosol, which falls within a predetermined selected size range, in the air introduced through the first inlet port 121.

Referring to FIGS. 5 and 6, the separation unit 200 includes a first separation unit configured to supply the airborne bioaerosol having a size range of 2.5 µm or less in the air through the first inlet port 121 and a second separation unit configured to supply the airborne bioaerosol having a size range of 10 µm or less in the air through the first inlet port 121, and at least one of the first and second separation units may be optionally installed.

Here, the airborne bioaerosol having a size range of 2.5 µm or less in the air may include airborne bacteria, and the airborne bioaerosol having a size range of 10 µm or less in the air may include airborne bacteria and airborne fungi.

Specifically, the airborne bioaerosol may be mainly divided into airborne bacteria having a size range of 2.5 µm or less and airborne fungi having a size range of more than 2.5 µm.

The airborne bacteria may include gram-negative bacteria and gram-positive bacteria. For example, the gram-negative bacteria may include Escherichia Coli having a size of approximately 0.91 µm, and the gram-positive bacteria may include Staphylococcus Epidermidis having a size of approximately 0.78 µm, and the like, but the present invention is not limited thereto.

Also, the airborne fungi may, for example, include mold, and the like, but the present invention is not limited thereto.

As such, the separation unit 200 may include first and second separation units 200a and 200b separately provided to collect the airborne bioaerosols having different size ranges, respectively.

The separation unit 200 includes an inflow unit 210 through which the outside air containing the airborne bioaerosol is introduced, a nozzle unit 220 configured to increase a flow velocity of the air flowing into the inflow unit, and an impingement unit 230 configured to impinge the bioaerosol included in the air passing through the nozzle unit 220 The inflow unit 210 has a second inlet port 211 through which the air containing the airborne bioaerosol is introduced.

Also, the nozzle unit 220 is fluidically connected to the inflow unit 210, and has at least one through hole 221 through which the introduced air flows.

The nozzle unit 220 has a cross-sectional area tapering from the inflow unit 210 toward the through hole 221, and thus may increase the flow velocity of the introduced air when the air is passing through the through hole 221.

Here, a plurality of through holes 221 may be provided. For example, three through holes 221 may be provided, but the present invention is not limited thereto.

The impingement unit 230 is fluidically connected to the nozzle unit 220, and includes an impingement plate 231 provided to impinge some of the bioaerosol included in the air introduced through the nozzle unit 220.

Here, the impingement plate 231 may be disposed to be spaced apart a predetermined distance from the through hole 221 of the nozzle unit.

The impingement unit 230 has an installation unit 233 installed on the first inlet port 121 side of the main body 120, and a second outlet port 232 provided to allow the air introduced through the nozzle unit 220 to be introduced into the first inlet port 121 of the main body 120 may be provided on the installation unit 223 side.

The impingement unit 230 may be provided to have at least a portion of the crosssectional area tapering from the impingement plate 231 side toward the installation unit 233 side. Therefore, because a flow velocity of the air introduced through the first inlet port 121 may increase, an amount of the bioaerosol attached to the swab may increase.

The installation unit 223 may be press-fitted to surround a portion of the lower end 1202b of the lower holder 1202 of the main body 120.

Because a sealing member 1207 (e.g., an O-ring) is provided at a portion of the lower end of the lower holder 1202 to which the installation unit 223 is attached, a space between the separation unit 200 and the main body 120 may be sealed more tightly.

Referring to FIG. 6, the impingement plate 231 has an opening 231a provided to allow relatively small particles to flow to the second outlet port 232 with small inertia.

The outside air containing the bioaerosol is accelerated through the through hole 221 of the nozzle unit so that a jet streamline is formed by the impingement plate 231. In this case, particles having a relatively large mass do not move along the jet streamline. Finally, only the particles having a size less than a certain size are allowed to pass through the opening 23 la and are discharged through the second outlet port 232.

The plurality of separation units 200 having a configuration as described above may be provided to collect the airborne bioaerosols having different size ranges, respectively, by adjusting a diameter d 1 of the through hole 221 of the nozzle unit and a distance d2 between the through hole 221 and the impingement plate 231.

For example, as the diameter d1 of the through hole 221 of the nozzle unit and the distance d2 between the through hole 221 and the impingement plate 231 become smaller, particles having a relatively smaller size may sequentially pass through the opening 231a and the first inlet port 121, and may be attached to a swab.

That is, the diameter dl of the through hole 221 of the nozzle unit and the distance d2 between the through hole 221 and the impingement plate 231 in the first separation unit may be provided to be smaller than the diameter d1 of the through hole 221 of the nozzle unit and the distance d2 between the through hole 221 and the impingement plate 231 in the second separation unit, respectively.

As described above, the first and second separation units have the same individual components, but may be provided to collect the airborne bioaerosols having different size ranges by adjusting the diameter d1 of the through hole 221 of the nozzle unit 220 and the distance d2 between the through hole 221 and the impingement plate 231.

That is, in the case of the above-described separation unit 200, the respective separation units (first and second separation units) formed by varying the diameter d1 of the through hole 221 and the distance d2 between the through hole 221 and the impingement plate 231 may be replaced and installed. Therefore, when collecting airborne bioaerosols having different size ranges, a plurality of swabs 110 may be replaced and installed to collect the airborne bioaerosol having different sizes.

For example, the first separation unit may be installed on the main body 120 to collect an airborne bioaerosol (for example, airborne bacteria) having a size range of 2.5 µm or less in the air using a swab (hereinafter referred to as a "first swab"), and the airborne bioaerosol may be separated from the first swab to measure an RLU value of the airborne bioaerosol using the ATP measurement unit 500 to be described below. Also, the second separation unit may be installed on the main body 120 to collect an airborne bioaerosol (for example, airborne bacteria and airborne fungi) having a size range of 10 µm or less in the air using a swab (hereinafter referred to as a "second swab"), and the airborne bioaerosol may be separated from the second swab to measure an RLU value of the airborne bioaerosol using the ATP measurement unit 500 to be described below.

That is, the ATP measurement unit 500 may measure RLU values of airborne bioaerosols including airborne bacteria and airborne bacteria and airborne fungi, respectively.

Meanwhile, the sampler 100 of the present invention includes an air flow device 300 configured to guide the flow of the air in order to attach the airborne bioaerosol onto the swab when the outside air containing the airborne bioaerosol is allowed to be introduced into an internal space of the main body 120.

The air flow device 300 is provided to form a pressure difference in order to allow the air to flow into the internal space of the main body 120. For example, a pump, a fan, or the like may be used to force the flow of the outside air flowing into the internal space of the main body 120.

The air flow device 300 may further include a flow rate control device 310 provided to adjust a flow rate of the introduced air under control of the air flow device 300.

The air flow device 300 and the flow rate control device 310 may be electrically connected.

The air flow device 300 is installed on the first outlet port 122 side of the main body to guide or force the outside air containing the airborne bioaerosol to flow toward the first outlet port 122 through the first inlet port 121 and the internal space in which the swab 110 is installed.

As such, the airborne bioaerosol introduced through the first inlet port 121 may be attached to the collection unit 111 of the swab.

In addition, the sampler 100 of the present invention further includes a humidification unit 400 provided to supply an aqueous solution containing a cell lysing agent toward the air flowing in the main body 120.

The humidification unit 400 includes a humidification nozzle unit 410 configured to spray an aqueous solution including a cell lysing agent toward the air flowing into the main body 120, and an aqueous solution supply unit (not shown) configured to supply the aqueous solution to the humidification nozzle unit 410.

More specifically, the humidification unit 400 is fluidically connected to the separation unit 200, and includes a humidification flow path 420 equipped with a third inlet port 421 through which the air containing the airborne bioaerosol is introduced.

Here, the aqueous solution supply unit provided to supply the aqueous solution to the humidification nozzle unit 410 may be configured separately as described above, and a space configured to accommodate the aqueous solution may be provided on the humidification nozzle unit 410. That is, the aqueous solution supply unit and the humidification nozzle unit may be integrally formed.

Also, the humidification unit 400 may further include a controller 430 configured to control the operation of the humidification nozzle unit 410, and the controller may control the operation of the humidification nozzle unit 410 using an On/Off switch.

In addition, the humidification nozzle unit 410 may be fluidically connected to at least a portion of the humidification flow path 420. For example, the humidification nozzle unit 410 may be installed at the humidification flow path 420 side to spray the aqueous solution containing a cell lysing agent toward the separation unit 200 connected to the humidification flow path 420.

Here, a configuration in which the humidification nozzle unit 410 is installed at the humidification flow path 420 has been described, but all types of configurations in which the humidification nozzle unit 410 may spray an aqueous solution containing a cell lysing agent toward the air supplied into the main body (toward the air supplied into the separation unit) are applicable.

The humidification nozzle unit 410 may atomize the cell lysing agent-containing aqueous solution along with humid air and supply the atomized aqueous solution to the separation unit 200 side.

For example, at least one selected from an NP-40 lysis buffer, a sodium dodecyl
sulfate (SDS) lysis buffer, a bacterial cell lysis buffer (Gold Biotechnology Inc.), a Triton based surfactant, and the like may be used as the cell lysing agent used in the cell lysing agent-containing aqueous solution.

Here, as the Triton-based surfactant, for example, Triton X-100 (Dow Chemical Inc.), which is a polyethylene P-T-octyl phenyl ether-based compound, may be used. More specifically, at least one selected from polyethylene glycol P-(1, 1,3,3-tetramethylbutyl)phenyl ether, octyl phenol ethoxylate, polyoxyethylene octyl phenyl ether, 4-octylphenol polyethoxylate, t-octylphenoxypolyethoxyethanol, and octoxynol-9 may be used in the present invention.

Because the humidification nozzle unit 410 is fluidically connected to the humidification flow path 420, when the air flowing into the third inlet port 421 is allowed to flow toward the separation unit 200, the humidification nozzle unit 410 may spray an aqueous solution toward the air flowing into the separation unit 200 so that both of the bioaerosol in the air and the cell lysing agent-containing aqueous solution can be allowed to flow into the separation unit 200.

That is, the bioaerosol in the air flowing in the third inlet port 421 may be allowed to
flow into the separation unit together with the cell lysing agent.

The cell lysing agent serves to lyse cell membranes of the airborne bioaerosol, and adenosine triphosphate (ATP) may be released from the airborne bioaerosol when the cell membranes are destroyed by the cell lysing agent.

Because such ATP reacts with a luminescent material as described below to emit light, the ATP is easily used to measure a concentration of the bioaerosol. That is, because the bioaerosol may be allowed to react with the cell lysing agent from the step of collecting the bioaerosol in the air in the humidification unit 400 according to the present invention, the time for extracting ATP from the bioaerosol may be shortened, thereby shortening the time for measuring the airborne bioaerosol.

Meanwhile, the measurement kit 10 of the present invention further includes the ATP measurement unit 500 configured to measure ATP in the airborne bioaerosol attached to the swab in order to measure a concentration of the airborne bioaerosol.

The ATP measurement unit 500 may be an RLU reader into which the collection unit 111 of the swab 110 is inserted to measure a relative luminescence unit (hereinafter referred to as "RLU") of the bioaerosol attached to the swab.

In addition, the ATP measurement unit 500 may further include an ATP monitor 510 configured to output each of the measured ATP values as an RLU (RLU/m³) value per unit air volume.

A method of optically measuring the bioaerosol may use a luminescent material and ATP released from the bioaerosol. The luminescent material includes luciferin and luciferase.

The luciferin is activated by ATP present in the lysed cells and converted into active luciferin, and the active luciferin is oxidized by an action of luciferase which is a luminescence enzyme and converted into oxyluciferin. In this case, chemical energy is converted into light energy to emit light.

Light emitted when the ATP extracted from the cells of the bioaerosol collected on the swab 110 reacts with the luminescent material may be measured using an RLU reader to calculate a concentration of the bioaerosol. In this case, ATP measurement unit 500 may further include a light source member (not shown) configured to irradiate light in order to facilitate measurement.

The measurement kit 10 including the ATP measurement unit 500 may shorten a measurement time because the measurement kit 10 may directly measure the bioaerosol collected on the swab 110.

Here, the ATP measurement unit 500 may include the ATP monitor 510 provided to output each of the measured ATP values as an RLU value.

Meanwhile, the measurement kit 10 of the present invention includes the particulate matter measurement unit 600 provided to measure a concentration of the particulate matter falling within a predetermined size range, which is included in the air (outside air).

The particulate matter measurement unit 600 may be provided to measure each of fine particulate matter (a particulate matter size of 10 µm or less (PM 10)) and ultrafine particulate matter (a particulate matter size of 2.5 µm or less (PM 2.5)) to output each of the measure values as a mass concentration value of the particulate matter per unit air volume (µg/m³)

In addition, referring to FIGS. 7 to 10, the measurement kit 10 of the present invention includes a control unit provided to receive first data output from the ATP measurement unit 500 and second data output from the particulate matter measurement unit 600 to generate third data.

More specifically, the control unit is provided to receive each of the first and second data to generate third data. In this case, because the control unit is electrically connected to each of the ATP measurement unit 500 and the particulate matter measurement unit 600 (is linked to transmit and receive the data), the control unit may be provided to receive the first and second data output from the respective measurement units to generate the third data converted through the control unit.

The control unit may further include a communication unit (not shown) provided to receive the first and second data output from the respective measurement units, and may generate third data based on the first and second data received from the communication unit.

The control unit may further include a user terminal capable of outputting a user interface, and may provide the generated third data to the user terminal.

Here, the communication unit may include short-range communication modules such as Wifi communication modules including a Bluetooth communication module, a Bluetooth low energy (BLE) communication module, a Zigbee communication module, a beacon communication module, and the like, an ultra-wideband (UWB) communication module, a LoRaWAN communication module, and the like, but the present invention is not limited thereto.

The user terminal may be provided to input user input values, and the user input values may, for example, include a volume flow rate of sampling air sampled using the sampler, a sampling time, a colony forming unit (CFU) conversion formula, a reference value for the airborne bioaerosol according to the measurement unit for each place, and reference values for fine particulate matter and ultrafine particulate matter for each place.

For example, the volume flow rate of the sampling air may refer to a volume (I/min) of the sampling air introduced per minute, the sampling time may refer to a sampling time in seconds (sec) required to collect the bioaerosol, the colony forming unit (CFU) conversion formula may refer to an correlation equation used to convert an RLU value into a CFU value, and the reference value may refers to a reference value for each of the airborne bioaerosols according to the concentration of the fine particulate matter or/and ultrafine particulate matter by place, which is judged by users.

As described above, the input user input values may be transmitted to the control unit.

Therefore, the control unit may generate third data based on the first and second data measured respectively from the ATP measurement unit and the particulate matter measurement unit, and the user input values selected from the above-described user input values.

Also, the user terminal may include a display device configured to output the user interface for the third data provided by the control unit.

For example, the user terminal may be a terminal that enables wired and wireless network communication. For example, the user terminal may include a smart phone, a portable multimedia player (PMP), personal digital assistants (PDAs), a desktop PC, a laptop PC, a tablet PC, and the like, but the present invention is not limited thereto.

That is, the control unit may estimate the airborne bioaerosol and the particulate matter (fine particulate matter and ultrafine particulate matter) measurement result data based on the first data, the second data, and the user input values received through the user terminal and provide a user interface, which represents the estimated measurement result data in various formats, to the user terminal. Also, the control unit may be composed of a server, a storage unit (not shown) configured to store and manage the data, and the like, in addition to the above-described communication unit.

Here, the first data includes RLU (RLU/m³) values of the airborne bioaerosol (for example, airborne bacteria) having a size of 2.5 µm or less and/or the airborne bioaerosol (for example, airborne bacteria and airborne fungi) having a size of 10 µm or less per unit air volume, and the second data includes mass concentration (µg/m³) values of the fine particulate matter (having a particulate matter size of 10 µm or less (PM 10) and/or the ultrafine particulate matter (having a particulate matter size of 2.5 µm or less (PM 2.5 ) per unit air volume.

Also, the control unit may generate concentration values between a concentration of the fine particulate matter and a concentration of the ultrafine particulate matter, that is, a mass concentration value of the particulate matter having a particle size of greater than 2.5 µm and less than 10 µm, based on each of the concentration (PM 10) of the fine particulate matter and the concentration (PM 2.5) of the ultrafine particulate matter measured through the particulate matter measurement unit, and transmit the generated concentration values to the user terminal.

Here, the values between the concentration of the fine particulate matter and the concentration of the ultrafine particulate matter may refer to values (PM 10 - PM 2.5) obtained by subtracting the concentration value of the ultrafine particulate matter from the concentration value of the fine particulate matter.

The third data may include at least one selected from an RLU value (RLU/µg [PM10]) of the airborne bioaerosol having a size range of 10 µm or less in the air per unit mass of the particulate matter calculated based on the first and second data or calculated based on the first data, the second data, and the user input values, an RLU value (RLU/µg [PM2.5]) of the airborne bioaerosol having a size range of 2.5 µm or less in the air per unit mass of the particulate matter, an RLU value (RLU/µg [PM10-PM2.5]) of the airborne bioaerosol having a size range of 2.5 µm to 10 µm in the air per unit mass of the particulate matter, a colony forming unit (CFU) value (CFU/µg [PM10]) of the airborne bioaerosol having a size range of 10 µm or less per unit mass of the particulate matter, a colony forming unit (CFU) value (CFU/µg [PM2.5]) of the airborne bioaerosol having a size range of 2.5 µm or less in the air per unit mass of the particulate matter, a colony forming unit (CFU) value (CFU/µg [PM10-PM2.5]) of the airborne bioaerosol having a size range of 2.5 µm to 10 µm in the air per unit mass of the particulate matter, a colony forming unit (CFU) value (CFU/m³[PM10]) of the airborne bioaerosol having a size range of 10 µm or less in the air per unit air volume, a colony forming unit (CFU) value (CFU/m³[PM2.5]) of the airborne bioaerosol having a size range of 2.5 µm or less in the air per unit air volume, a colony forming unit (CFU) value (CFU/m³[PM10-PM2.5]) of the airborne bioaerosol having a size range of 2.5 µm to 10 µm in the air per unit air volume, and a mass concentration value (µg/m³) of the particulate matter having a particle size of greater than 2.5 µm and less than 10 µm per unit air volume.

Here, the third data is related to cases where the airborne bioaerosol (PM 10) having a size range of 10 µm or less in the air includes both airborne bacteria and airborne fungi, the airborne bioaerosol (PM2.5) having a size range of 2.5 µm or less in the air include airborne bacteria (a size of the airborne bacteria is mainly less than or equal to 2 micrometers (µm)), and the airborne bioaerosol (PM10-PM2.5) having a size range of 2.5 µm to 10 µm in the air mainly includes airborne fungi.

Because the size of the airborne fungi is mainly greater than or equal to 3 µm, the airborne bioaerosol having a size range of 2.5 µm to 10 µm in the air may include some of the airborne bacteria, but mainly includes airborne fungi.

The colony forming unit (CFU) value may be deduced using the colony forming unit conversion formula.

The RLU and CFU have a linear relationship in the colony forming unit (CFU) conversion formula.

As described above, each of the plurality of pieces of third data generated from the measurement results based on the first data, the second data, and the user input values may be provided to the user terminal to easily determine the number of airborne fungi or/and airborne bacteria according to the concentrations of the fine particulate matter and ultrafine particulate matter at measurement places for each unit required by users without any separate comparison between the first data and the second data.

In particular, the user interface may include a user input window 1000 configured to input at least one selected from a volume flow rate of the sampling air, a sampling time, a colony forming unit (CFU) conversion formula, a reference value for the airborne bioaerosol per mass or volume of the fine particulate matter, and a reference value for the airborne bioaerosol per mass or volume of the ultrafine particulate matter; a data input window (not shown) configured to transmit the first and second data to the control unit; a third data output window 2000 configured to provide a numerical value of the airborne bioaerosol according to the location of the measurement result data, and the concentrations of the fine particulate matter and ultrafine particulate matter, and a third data schematization output window 3000 configured to provide each piece of the third data in a schematized form.

The user input window 1000 includes a sampling air volume flow rate input window 1001, a sampling time input window 1002, a colony forming unit (CFU) conversion formula input window 1003, and a reference value input window 1004 configured to input a reference value for the airborne bioaerosol per mass or volume of the fine particulate matter and a reference value for the airborne bioaerosol per mass or volume of the ultrafine particulate matter. In this case, each of the numerical values may be input into the user interface output through the user terminal, and the numerical values in the input window may be optionally input according to the users' purpose of analysis.

Here, each of the reference values input through the reference value input window 1004 may be, for example, each of reference values for mass concentrations and/or CFU values of the airborne bioaerosol (10 µm or less, 2.5 µm or less, and 2.5 µm to 10 µm) per unit air volume at a certain place, and each of reference values for RLU values and/or CFU values per unit mass of the airborne bioaerosol.

In particular, when a user input window which is not required to estimate the measurement result data is present depending on the users, a user may manipulate a management option (not shown) of the user input window to display only the necessary user input windows on the user interface.

For example, when a user is required to check the RLU values rather than the CFU values, the user may not input a separate numerical value into the colony forming unit (CFU) conversion formula input window, or may manipulate the management option of the user input window so as not to display the colony forming unit (CFU) conversion formula input window on the user interface.

In addition, the third data output window 2000 includes each of measurement place output windows 2001 and RLU numerical value output windows 2002 for the airborne bioaerosol having a size of 10 µm or less and the airborne bioaerosol having a size of 2.5 µm or less for each place.

For example, the RLU numerical value output window 2002 for the airborne bioaerosol for each place may output the RLU numerical value for the airborne bacteria and airborne fungi and the RLU numerical value for the airborne bacteria.

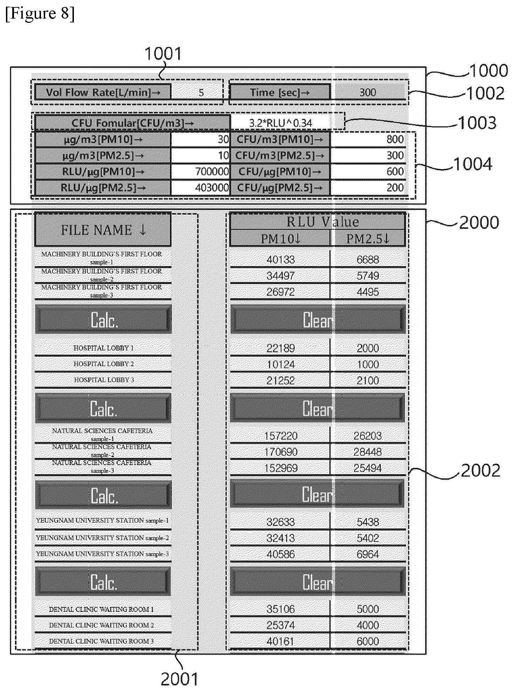

As described above, when each of the numerical values is input through the user input window 1000 according to the purpose of analysis, and the first and second data measured through each of the measurement units 500 and 600 are received by the control unit, as shown in FIG. 8, the places at which the bioaerosol (airborne fungi and airborne bacteria, or airborne germs) are measured may be displayed on the measurement place output window 2001, and each of the RLU numerical values and/or CFU numerical values measured for each of the places may be displayed on the RLU numerical value output window 2002.

In addition, the third data schematization output window 3000 may schematize and output the plurality of pieces of third data in the form of a bar graph, a broken line graph, a radar graph, a pie graph, or the like in order to provide the user's intuitive analysis of the plurality of pieces of third data, and may output numerical values regarding each of the reference values input through the reference value input window 1004 together with the schematized data.

Here, the third data schematization output window 3000 includes an ATP output window 3001 configured to output each of the RLU numerical values of the airborne bioaerosol per unit air volume by measurement place in the above-described schematized form for the first data on the user interface, and a PM output window 3002 configured to output the mass concentration values of the fine particulate matter (PM20) and ultrafine particulate matter (PM2.5) per unit air volume by measurement place in the above-described schematized form for the second data on the user interface.

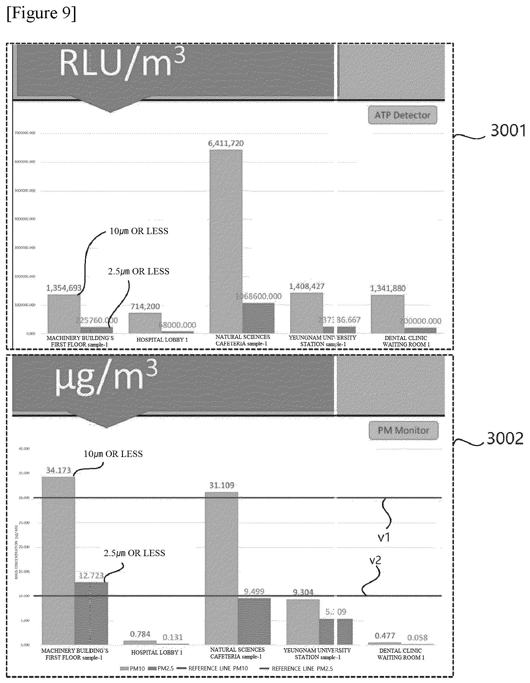

For example, referring to FIG. 9, the ATP output window 3001 may output the RLU numerical values of the airborne bioaerosol (including airborne fungi and airborne bacteria) having a size range of 10 µm or less per unit air volume by measurement place and the airborne bioaerosol (including airborne bacteria) having a size range of 2.5 µm or less in the air for the first data in the form of respective bar graphs on the user interface, the PM output window 3002 may output the mass concentration values of the fine particulate matter (PM10) and ultrafine particulate matter (PM2.5) per unit air volume by measurement place for the second data in the form of respective bar graphs.

Here, a reference line v representing each of the reference values for the airborne bioaerosol and/or the reference values for the fine particulate matter and ultrafine particulate matter according to the size range by measurement place may be displayed in the form of a horizontal straight line on each of the output windows.

The reference line v includes a PM10 reference line v1 representing a reference value for fine particulate matter, and a PM2.5 reference line v2 representing a reference value for ultrafine particulate matter. Also, each of a first reference line v3 representing a reference value for the airborne bioaerosol (including airborne fungi and airborne bacteria) having a size range of 10 µm or less according to each of the units, a second reference line v4 representing a reference value for the airborne bioaerosol (including airborne bacteria) having a size range of 2.5 µm or less in the air, and a third reference line (not shown) representing a reference value for the airborne bioaerosol (mainly including airborne fungi) having a size range of 2.5 µm to 10 µmin air may be displayed in the form of a horizontal straight line.

Also, the third data schematization output window 3000 includes a first output window 3003 configured to output the RLU numerical values of the airborne bioaerosol (including airborne fungi and airborne bacteria) having a size range of 10 µm or less per mass of the unit particulate matter (fine particulate matter and ultrafine particulate matter) by place for the third data, the airborne bioaerosol (including airborne bacteria) having a size range of 2.5 µm or less in the air, and the airborne bioaerosol (mainly including airborne fungi) having a size range of 2.5 µm to 10 µm in the air in the above-described schematized form on the user interface.

Also, the third data schematization output window 3000 includes a second output window 3004 configured to output the CFU numerical values of the airborne bioaerosol (including airborne fungi and airborne bacteria) having a size range of 10 µm or less per air volume of the unit particulate matter (fine particulate matter and ultrafine particulate matter) by place, the airborne bioaerosol (including airborne bacteria) having a size range of 2.5 µm or less in the air, and the airborne bioaerosol (mainly including airborne fungi) having a size range of 2.5 µm to 10 µmin the air in the above-described schematized form.

Furthermore, the third data schematization output window 3000 includes a third output window 3005 configured to output the CFU numerical values of the airborne bioaerosol (including airborne fungi and airborne bacteria) having a size range of 10 µm or less per mass of the unit particulate matter (fine particulate matter and ultrafine particulate matter) by place, the airborne bioaerosol (including airborne bacteria) having a size range of 2.5 µm or less in the air, and the airborne bioaerosol (mainly including airborne fungi) having a size range of 2.5 µm to 10 µmin the air in the above-described schematized form.

In particular, the third data schematization output window 3000 may further include a fourth output window (not shown) configured to output a mass concentration value of the particulate matter having a particle size range between the fine particulate matter (PM10) and the ultrafine particulate matter (PM2.5) per unit air volume by measurement place in the form of a bar graph.

That is, the fourth output window may output each of the mass concentration values (µg/m³) of the particulate matter having a particle size of greater than 2.5 µm and less than 10 µm per unit air volume by place in the above-described schematized form.

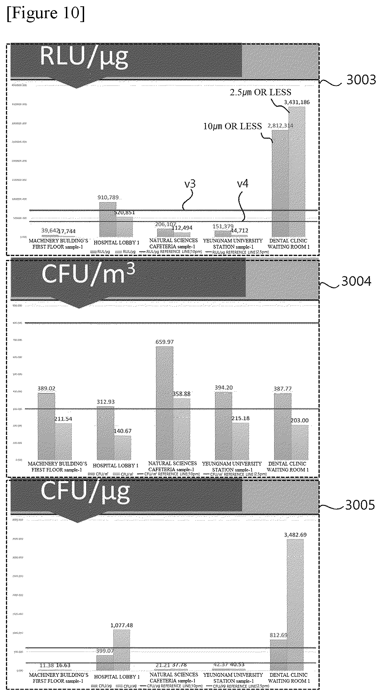

For example, referring to FIG. 10, the first output window 3003 may output each of the RLU numerical values of the airborne bioaerosol (including airborne fungi and airborne bacteria) having a size range of 10 µm or less per mass of the unit particulate matter (fine particulate matter and ultrafine particulate matter) by place, the airborne bioaerosol (including airborne bacteria) having a size range of 2.5 µm or less in the air, and the airborne bioaerosol (mainly including airborne fungi) having a size range of 2.5 µm to 10 µmin the air in the form of a bar graph.

Here, the RLU numerical value of the airborne bioaerosol (mainly including airborne fungi) having a size range of 2.5 µm to 10 µm in the air may refer to a value obtained by subtracting a measured value of the airborne bioaerosol having a size range of 2. 5 µm or less from a measured value of the airborne bioaerosol having a size range of 10 µm or less.

Also, the second output window 3004 may output each of the CFU numerical values of the airborne bioaerosol (including airborne fungi and airborne bacteria) having a size range of 10 µm or less per air volume of the unit particulate matter (fine particulate matter and ultrafine particulate matter) by place, the airborne bioaerosol (including airborne bacteria) having a size range of 2.5 µm or less in the air, and the airborne bioaerosol (mainly including airborne fungi) having a size range of 2.5 µm to 10 µmin the air in the form of a bar graph, and the third output window 3005 may output each of the CFU numerical values of the airborne bioaerosol (including airborne fungi and airborne bacteria) having a size range of 10 µm or less per mass of the unit particulate matter (fine particulate matter and ultrafine particulate matter) by place, the airborne bioaerosol (including airborne bacteria) having a size range of 2.5 µm or less in the air, and the airborne bioaerosol (mainly including airborne fungi) having a size range of 2.5 µm to 10 µmin the air in the form of a bar graph.

Here, the first reference line v 1, the second reference line v2, and the third reference line (not shown) representing a reference value for the airborne bioaerosol (including airborne fungi and airborne bacteria) having a size range of 10 µm or less according to each of the units, a reference value for the airborne bioaerosol (including airborne bacteria) having a size range of 2.5 µm or less in the air, and a reference value for the airborne bioaerosol (mainly including airborne fungi) having a size range of 2.5 µm to 10 µm in the air, respectively, may be displayed in the form of a horizontal straight line on each of the output windows.

Therefore, because each of the pieces of data on the concentration of the fine particulate matter and ultrafine particulate matter, the concentration of the airborne bacteria and airborne fungi, the concentration of the airborne bacteria, and the concentration of the airborne fungi for each place may be directly compared with the reference value, a relative proportion of the bacterial and fungal particles may be more easily and rapidly determined.

Meanwhile, referring to FIG. 11, a sampler 100a according to a second embodiment of the present invention includes at least one swab 110, a main body 120, a first separation unit 200a, a second separation unit 200b, first and second flow paths 251 and 252, a valve 253, and an air flow device 300.

Hereinafter, a description of the same components as those of the sampler 100 according to the first embodiment as described above will be omitted. In the sampler 100 according to the first embodiment of the present invention, both of the swab 110 and the separation unit 200 are replaced and installed to separate and collect airborne bioaerosols having different size ranges. However, in the sampler 100a according to the second embodiment, because each of the first and second separation units 200a and 200b is connected to one main body 120 unlike the first embodiment, only the swab 110 may be replaced to separate and collect the airborne bioaerosols having different size ranges.

More particularly, the sampler 100a according to the second embodiment includes first and second flow paths 251 and 252 fluidically connected to the first inlet port 121 of the main body 120, respectively.

Also, the first separation unit 200a is installed on the first flow path 251 side, and is provided to supply the airborne bioaerosol falling within a first size range in the air introduced through the first inlet port 121.

Also, the second separation unit 200b is installed on the second flow path 252 side, and is provided to supply the airborne bioaerosol falling within a second size range in the air introduced through the first inlet port 121.

Here, the airborne bioaerosol falling within the first size range may have a size range of 2.5 µm or less in the air, and the airborne bioaerosol falling within the second size range may have a size range of 10 µm or less in the air.

In addition, the valve 253 may be provided on the first inlet port 121 side to fluidically connect the first inlet port 121 to the first flow path 251, or may be provided to fluidically connect the first inlet port 121 to the second flow path 252.

For example, the valve 253 may be controlled so that the first inlet port 121 can be fluidically connected to the first flow path 251. In this case, when the airborne bioaerosol falling within the first size range is attached to the swab 110 and collected, the swab to which the airborne bioaerosol falling within the first size range is attached is detached from the main body. Then, another swab is installed, and the valve 253 is controlled again to fluidically connect the first inlet port 121 to the second flow path 252. In this case, the airborne bioaerosol falling within the second size range may be attached to another swab to collect the airborne bioaerosol.

Here, the valve 253 may be, for example, a three-way valve, but the present invention is not limited thereto.

Also, the air flow device forces the flow of the outside air into the main body.

Meanwhile, referring to FIG. 12, a sampler 100b according to a third embodiment of the present invention includes first and second swabs 110a and 110b, first and second main bodies 120a and 120b, first and second separation units 200a and 200b, and an air flow device 300.

Hereinafter, a description of the same components as those of the samplers 100 and 100b according to the first or/and second embodiments as described above will be omitted.

In the sampler 100 according to the first embodiment of the present invention, both of the swab 110 and the separation unit 200 are replaced and installed to separate and collect airborne bioaerosols having different size ranges. However, in the sampler 100b according to the third embodiment, because the first and second swabs 110a and 110b are respectively installed in the first and second main bodies 120a and 120b and are respectively connected to the first and second separation units 200a and 200b unlike the first embodiment, the airborne bioaerosols having different size ranges may be separated and collected without replacing the swab 110 and the separation unit.

More particularly, the sampler 100b according to the third embodiment may have a form in which the samplers 100 according to the first embodiment are connected as a pair. The sampler 100b according to the third embodiment includes first and second swabs 110a and 110b provided to attach an airborne bioaerosol thereto.

Also, the sampler 100b according to the third embodiment includes first and second main bodies 120a and 120b, each of which has an internal space, wherein each of the first and second swabs 110a and 110b is installed in the internal space, and having first inlet ports 121a and 121b and a first outlet port (not shown) provided, respectively, to allow outside air containing the airborne bioaerosol to be introduced and discharged.

Also, the sampler 100b according to the third embodiment includes a first separation unit 200a installed at the first main body 120a and provided to supply the airborne bioaerosol falling within a first size range in the air introduced through the first inlet port 121 a of the first main body, and a second separation unit 200b installed at the second main body 120b and provided to supply the airborne bioaerosol falling within a second size range in the air introduced through the first inlet port 121b of the second main body.

Also, the sampler 100b according to the third embodiment includes an air flow device (not shown) configured to guide the outside air containing the airborne bioaerosol to flow toward the first outlet port (not shown) through the first inlet ports 121a and 121b and the internal space in which the swabs 110a and 110b are installed.

Here, the air flow device forces the flow of the outside air into the main body.

Also, the air flow device may be provided to be connected to each of the first outlet ports, and one air flow device may be connected to each of the first outlet ports, and may be provided to guide the flow of the air into the first main body and the second main body.

Further, the present disclosure provides a method of measuring an airborne bioaerosol and particulate matter.

For example, the method of measuring an airborne bioaerosol and particulate matter relates to a method of measuring an airborne bioaerosol and particulate matter using the measurement kit 10 including the sampler according to the first to third embodiments as described above. Therefore, the contents disclosed for the measurement kit 10 may be equally applied to the specific contents of a device for measuring an airborne bioaerosol and particulate matter as will be described below.

First, the method of measuring an airborne bioaerosol and particulate matter using the measurement kit 10 including the sampler according to the first embodiment as described above includes a bioaerosol concentration measurement step of measuring ATP in the airborne bioaerosol attached to a swab; a particulate matter concentration measurement step of measuring a concentration of the particulate matter; and a data outputting step of receiving first data output from the ATP measurement unit and second data output from the particulate matter measurement unit to output third data from the control unit.

In addition, the method of measuring an airborne bioaerosol and particulate matter using the measurement kit 10 including the sampler according to the second embodiment as described above includes a bioaerosol concentration measurement step of measuring ATP in the airborne bioaerosol attached to a swab; a particulate matter concentration measurement step of measuring a concentration of the particulate matter concentration; and a data outputting step of receiving first data output from the ATP measurement unit and second data output from the particulate matter measurement unit to output third data from the control unit.

Further, the method of measuring an airborne bioaerosol and particulate matter using the measurement kit 10 including the sampler according to the third embodiment as described above includes a bioaerosol concentration measurement step of measuring ATP in the airborne bioaerosol attached to each of the swabs; a particulate matter concentration measurement step of measuring a concentration of the particulate matter; and a data outputting step of receiving first data output from the ATP measurement unit and second data output from the particulate matter measurement unit to output third data from the control unit.

## Claims

1. A kit (10) for measuring an airborne bioaerosol and particulate matter, comprising:
a sampler (100; 100a; 100b) comprising at least one swab (110; 110a, 110b) provided to attach an airborne bioaerosol thereto; a main body (120) having an internal space, wherein a swab (110; 110a; 110b) of the at least one swab is detachably installed in the internal space, and having a first inlet port (121) and a first outlet port (122) provided, respectively, to allow outside air to be introduced and discharged; at least one separation unit (200; 200a, 200b) detachably installed on the first inlet port (121) side of the main body (120) and provided to supply the airborne bioaerosol, which falls within a predetermined selected size range, in the air introduced through the first inlet port (121); and an air flow device (300) installed on the first outlet port (122) side of the main body (120) to guide the outside air to flow toward the first outlet port (122) through the first inlet port (121) and the internal space in which the swab (110; 110a, 110b) is installed by forming a pressure difference in order to allow the air to flow into the internal space of the at least one main body (120); and
an adenosine triphosphate, ATP, measurement unit (500) provided to measure ATP in the airborne bioaerosol attached to the swab (110; 110a, 110b);
**characterized in that**
the kit further comprises a humidification unit (400) provided to supply an aqueous solution containing a cell lysing agent toward the air flowing into the main body (120);
a particulate matter measurement unit (600) provided to measure a concentration of the particulate matter, which falls within a predetermined size range, included in the air; and
a control unit provided to receive first data output from the ATP measurement unit (500) and second data output from the particulate matter measurement unit (600) to generate third data based on at least one of the first and second data.

2. The kit (10) of claim 1, where the sampler further comprises each of first and second flow paths (251, 252) fluidically connected to the first inlet port (121); and a valve (253) provided on the first inlet port (121) side and provided to fluidically connect the first inlet port (121) to the first flow path (251) or fluidically connect the first inlet port (121) to the second flow path (252),
wherein the at least one separation unit (200) comprise a first separation unit (200a) detachably installed on the first flow path (251) side and provided to supply the airborne bioaerosol, which falls within a first predetermined size range, in the air introduced through the first inlet port (121); a second separation unit (200b) detachably installed on the second flow path (252) side of and provided to supply airborne bioaerosol, which falls within a second predetermined size range, in the air introduced through the first inlet port (121).

3. The kit (10) of claim 2, wherein the airborne bioaerosol falling within the first predetermined size range has a size range of 2.5 µm or less in the air, and
the airborne bioaerosol falling within the second predetermined size range has a size range of 10 µm or less in the air.

4. The kit (10) of claim 1, wherein the at least one separation unit (200; 200a, 200b) comprises a first separation unit (200a) configured to supply the airborne bioaerosol having a size range of 2.5 µm or less in the air through the first inlet port (121) and a second separation unit (200b) configured to supply the airborne bioaerosol having a size range of 10 µm or less in the air through the first inlet port (121), and at least one of the first and second separation units (200a, 200b) is installed.

5. The kit (10) of claim 1, wherein the particulate matter measurement unit (600) is configured to measure each of particulate matter of a particulate matter size of 10 µm or less, PM 10, and particulate matter of a particulate matter size of 2.5 µm or less, PM 2.5, to output each of the measure values as a mass concentration value of the particulate matter per unit air volume.

6. The kit (10) of claim 1, wherein the ATP measurement unit (500) further comprises an ATP monitor (510) configured to output each of the measured ATP values as a relative luminescence unit, RLU, value per unit air volume.

7. The kit (10) of claim 1, further comprising a user terminal configured to output a user interface, wherein the control unit is configured to provide the generated third data to the user terminal.

8. The kit (10) of claim 7, wherein the user terminal is provided to input user input values, and the user input values comprise at least one selected from a volume flow rate of sampling air, a sampling time, a colony forming unit, CFU, conversion formula, a reference value for the airborne bioaerosol per mass or volume of the particulate matter of a particulate matter size of 10 µm or less for each measurement place, and a reference value for the airborne bioaerosol per mass or volume of the particulate matter of a particulate matter size of 2.5 µm or less for each measurement place.

9. The kit (10) of claim 8, wherein the control unit is configured to generate the third data based on the user input values and at least one of the first data and the second data.

10. The kit (10) of claim 9, wherein the third data comprises at least one selected from an RLU value of the airborne bioaerosol having a size range of 10 µm or less in the air per unit mass of the particulate matter, RLU/µg [PM10], an RLU value of the airborne bioaerosol having a size range of 2.5 µm or less in the air per unit mass of the particulate matter, RLU/µg [PM2.5] , an RLU value of the airborne bioaerosol having a size range of 2.5 µm to 10 µm in the air per unit mass of the particulate matter, RLU/µg [PM10-PM2.5], a colony forming unit, CFU value of the airborne bioaerosol having a size range of 10 µm or less per unit mass of the particulate matter, CFU/µg [PM10], a colony forming unit, CFU value of the airborne bioaerosol having a size range of 2.5 µm or less in the air per unit mass of the particulate matter, CFU/µg [PM2.5],, a colony forming unit, CFU value of the airborne bioaerosol having a size range of 2. 5 µm to 10 µm in the air per unit mass of the particulate matter, CFU/µg [PM10- PM2.5],, a colony forming unit, CFU value of the airborne bioaerosol having a size range of 10 µm or less in the air per unit air volume, CFU/m³[PM10], a colony forming unit, CFU value of the airborne bioaerosol having a size range of 2.5 µm or less in the air per unit air volume CFU/m³[PM2.5], a colony forming unit, CFU value of the airborne bioaerosol having a size range of 2.5 µm to 10 µm in the air per unit air volume CFU/m³[PM10-PM2.5], and a mass concentration value, µg/m³, of the particulate matter having a particle size of greater than 2.5 µm and less than 10 µm per unit air volume.

11. The kit (10) of claim 7, wherein the user interface further comprises a user input window (1000) configured for inputting at least one user input value, a third data output window (2000) configured to output the generated third data, and a third data schematization output window (3000) configured to provide each piece of the third data in a schematized form.

12. The kit (10) of claim 11, wherein
the third data schematization output window (3000)is configured to display, in the form of a horizontal straight line (v1, v2, v3, v4), each of reference lines representing a reference value for the airborne bioaerosol and/or reference values for particulate matter of a of size of 10 µm or less and particulate matter of a of size of 2.5 µm or less for each measurement place based on the user input values.

13. The kit of claim 1, wherein the at least one swab (110) comprises first and second swabs (110a, 110b), wherein the main body (120) is a first main body, wherein the first swab is detachably installed in the internal space of the first main body and the sampler further comprises a second main body having an internal space,
wherein the second swab is detachably installed in the internal space of the second main body, and having a first inlet port (121a, 121b) and a first outlet port (122) provided, respectively, to allow outside air to be introduced and discharged;
wherein the at least one separation unit comprise a first separation unit (200a) detachably installed on the first main body (120a) and provided to supply the airborne bioaerosol, which falls within a first predetermined size range, in the air introduced through the first inlet port (121a) of the first main body (121a); a second separation unit (200b) detachably installed on the second main body (120b) and provided to supply the airborne bioaerosol, which falls within a second predetermined size range, in the air introduced through the first inlet port (121b) of the second main body (120b), and wherein the air flow device (300) is connected to each of the first outlet ports and is configured to guide the outside air to flow toward the first outlet ports through the first inlet ports and the internal spaces in which the swabs are installed or one air flow device is connected to each of the first outlet ports and is configured to guide the flow of air into the first main body and the second main body.

## Patentansprüche

1. Kit (10) zur Messung eines in der Luft vorhandenen Bioaerosols und von Partikeln, umfassend:
einen Probennehmer (100; 100a; 100b), der mindestens einen Tupfer (110; 110a, 110b) umfasst, der dazu vorgesehen ist, dass ein in der Luft vorhandenes Bioaerosol daran anhaftet; einen Hauptkörper (120) mit einem Innenraum, wobei ein Tupfer (110; 110a; 110b) des mindestens einen Tupfers lösbar in dem Innenraum installiert ist, und mit einer ersten Einlassöffnung (121) und einer ersten Auslassöffnung (122), die jeweils dazu vorgesehen sind, das Einführen bzw. Ausleiten von Außenluft zu ermöglichen; mindestens eine Trenneinheit (200; 200a, 200b), die lösbar auf der Seite der ersten Einlassöffnung (121) des Hauptkörpers (120) installiert ist und dazu vorgesehen ist, das in einen vorbestimmten ausgewählten Größenbereich fallende, in der durch die erste Einlassöffnung (121) eingeführten Luft vorhandene Bioaerosol zuzuführen; und eine Luftfördervorrichtung (300), die auf der Seite der ersten Auslassöffnung (122) des Hauptkörpers (120) installiert ist, um die Außenluft durch die erste Einlassöffnung (121) und den Innenraum, in dem der Tupfer (110; 110a, 110b) installiert ist, in Richtung der ersten Auslassöffnung (122) zu führen, indem ein Druckunterschied erzeugt wird, damit die Luft in den Innenraum des mindestens einen Hauptkörpers (120) strömen kann; und
eine Adenosintriphosphat- (ATP-)Mess-Einheit (500), die dazu vorgesehen ist, ATP in dem am Tupfer (110; 110a, 110b) anhaftenden, in der Luft vorhandenen Bioaerosol zu messen;
**dadurch gekennzeichnet, dass**
das Kit ferner eine Befeuchtungseinheit (400) umfasst, die dazu vorgesehen ist, eine wässrige Lösung, die ein Zelllyse-Mittel enthält, zu der in den Hauptkörper (120) strömenden Luft zuzuführen;
eine Partikelmess-Einheit (600), die dazu vorgesehen ist, eine Konzentration der in einem vorbestimmten Größenbereich liegenden, in der Luft enthaltenen Partikel zu messen; und
eine Steuereinheit, die dazu vorgesehen ist, erste Daten, die von der ATP-Mess-Einheit (500) ausgegeben werden, und zweite Daten, die von der Partikelmess-Einheit (600) ausgegeben werden, zu empfangen, um dritte Daten auf Basis von mindestens einem der ersten und zweiten Daten zu erzeugen.

2. Kit (10) nach Anspruch 1, wobei der Probennehmer jeweils erste und zweite Strömungspfade (251, 252) umfasst, die fluidisch mit der ersten Einlassöffnung (121) verbunden sind; und ein Ventil (253), das auf der Seite der ersten Einlassöffnung (121) vorgesehen ist und dazu vorgesehen ist, die erste Einlassöffnung (121) fluidisch mit dem ersten Strömungspfad (251) zu verbinden oder die erste Einlassöffnung (121) fluidisch mit dem zweiten Strömungspfad (252) zu verbinden,
wobei die mindestens eine Trenneinheit (200) eine erste Trenneinheit (200a) umfasst, die lösbar auf der Seite des ersten Strömungspfads (251) installiert ist und dazu vorgesehen ist, das in einen ersten vorbestimmten Größenbereich fallende, in der durch die erste Einlassöffnung (121) eingeführten Luft vorhandene Bioaerosol zuzuführen; und eine zweite Trenneinheit (200b), die lösbar auf der Seite des zweiten Strömungspfads (252) installiert ist und dazu vorgesehen ist, das in einen zweiten vorbestimmten Größenbereich fallende, in der durch die erste Einlassöffnung (121) eingeführten Luft vorhandene Bioaerosol zuzuführen.

3. Kit (10) nach Anspruch 2, wobei das in den ersten vorbestimmten Größenbereich fallende, in der Luft vorhandene Bioaerosol einen Größenbereich von 2,5 µm oder weniger in der Luft aufweist, und das in den zweiten vorbestimmten Größenbereich fallende, in der Luft vorhandene Bioaerosol einen Größenbereich von 10 µm oder weniger in der Luft aufweist.

4. Kit (10) nach Anspruch 1, wobei die mindestens eine Trenneinheit (200; 200a, 200b) eine erste Trenneinheit (200a) umfasst, die dazu konfiguriert ist, das in der Luft vorhandene Bioaerosol mit einem Größenbereich von 2,5 µm oder weniger durch die erste Einlassöffnung (121) zuzuführen, und eine zweite Trenneinheit (200b), die dazu konfiguriert ist, das in der Luft vorhandene Bioaerosol mit einem Größenbereich von 10 µm oder weniger durch die erste Einlassöffnung (121) zuzuführen, und wobei mindestens eine der ersten und zweiten Trenneinheiten (200a, 200b) installiert ist.

5. Kit (10) nach Anspruch 1, wobei die Partikelmess-Einheit (600) dazu konfiguriert ist, jeweils Partikel mit einer Partikelgröße von 10 µm oder weniger (PM10) und Partikel mit einer Partikelgröße von 2,5 µm oder weniger (PM2,5) zu messen, um jeweils die Messwerte als Massenkonzentrationswert der Partikel pro Volumeneinheit Luft auszugeben.

6. Kit (10) nach Anspruch 1, wobei die ATP-Mess-Einheit (500) zusätzlich einen ATP-Monitor (510) umfasst, der dazu konfiguriert ist, jeweils die gemessenen ATP-Werte als Relative-Lumineszenzeinheit- (RLU-)Werte pro Volumeneinheit Luft auszugeben.

7. Kit (10) nach Anspruch 1, ferner umfassend ein Benutzerterminal, das dazu konfiguriert ist, eine Benutzeroberfläche auszugeben, wobei die Steuereinheit dazu konfiguriert ist, die erzeugten dritten Daten dem Benutzerterminal bereitzustellen.

8. Kit (10) nach Anspruch 7, wobei das Benutzerterminal dazu vorgesehen ist, Benutzereingabewerte einzugeben, und die Benutzereingabewerte mindestens eine Auswahl aus einem Volumenstrom der Probenahmeluft, einer Probenahmezeit, einer Umrechnungsformel für kolonie-bildende Einheiten (CFU), einem Referenzwert für das in der Luft vorhandene Bioaerosol pro Masse oder Volumen der Partikel mit einer Partikelgröße von 10 µm oder weniger für jeden Messort und einem Referenzwert für das in der Luft vorhandene Bioaerosol pro Masse oder Volumen der Partikel mit einer Partikelgröße von 2,5 µm oder weniger für jeden Messort umfassen.

9. Kit (10) nach Anspruch 8, wobei die Steuereinheit dazu konfiguriert ist, die dritten Daten basierend auf den Benutzereingabewerten und mindestens einem der ersten Daten und der zweiten Daten zu erzeugen.

10. Kit (10) nach Anspruch 9, wobei die dritten Daten mindestens eine Auswahl aus einem RLU-Wert des in der Luft vorhandenen Bioaerosols mit einem Größenbereich von 10 µm oder weniger pro Masseneinheit der Partikel (RLU/µg [PM10]), einem RLU-Wert des in der Luft vorhandenen Bioaerosols mit einem Größenbereich von 2,5 µm oder weniger pro Masseneinheit der Partikel (RLU/µg [PM2,5]), einem RLU-Wert des in der Luft vorhandenen Bioaerosols mit einem Größenbereich von 2,5 µm bis 10 µm pro Masseneinheit der Partikel (RLU/µg [PM10-PM2,5]), einem CFU-Wert des in der Luft vorhandenen Bioaerosols mit einem Größenbereich von 10 µm oder weniger pro Masseneinheit der Partikel (CFU/µg [PM10]), einem CFU-Wert des in der Luft vorhandenen Bioaerosols mit einem Größenbereich von 2,5 µm oder weniger pro Masseneinheit der Partikel (CFU/µg [PM2,5]), einem CFU-Wert des in der Luft vorhandenen Bioaerosols mit einem Größenbereich von 2,5 µm bis 10 µm pro Masseneinheit der Partikel (CFU/µg [PM10-PM2,5]), einem CFU-Wert des in der Luft vorhandenen Bioaerosols mit einem Größenbereich von 10 µm oder weniger pro Volumeneinheit Luft (CFU/m³ [PM10]), einem CFU-Wert des in der Luft vorhandenen Bioaerosols mit einem Größenbereich von 2,5 µm oder weniger pro Volumeneinheit Luft (CFU/m³ [PM2,5]), einem CFU-Wert des in der Luft vorhandenen Bioaerosols mit einem Größenbereich von 2,5 µm bis 10 µm pro Volumeneinheit Luft (CFU/m³ [PM10-PM2,5]) sowie einem Massenkonzentrationswert (µg/m³) der Partikel mit einer Partikelgröße von größer als 2,5 µm und kleiner als 10 µm pro Volumeneinheit Luft umfasst.

11. Kit (10) nach Anspruch 7, wobei die Benutzeroberfläche ferner ein Benutzereingabefenster (1000) zum Eingeben von mindestens einem Benutzereingabewert, ein Ausgabefenster für dritte Daten (2000) zum Ausgeben der erzeugten dritten Daten und ein Schematisierungs-Ausgabefenster für dritte Daten (3000) zum Bereitstellen jeweils eines Teils der dritten Daten in schematisierter Form umfasst.

12. Kit (10) nach Anspruch 11, wobei das Schematisierungs-Ausgabefenster für dritte Daten (3000) dazu konfiguriert ist, jeweils Referenzlinien (v1, v2, v3, v4), die einen Referenzwert für das in der Luft vorhandene Bioaerosol und/oder Referenzwerte für Partikel mit einer Größe von 10 µm oder weniger und Partikel mit einer Größe von 2,5 µm oder weniger für jeden Messort darstellen, in Form einer horizontalen Geraden basierend auf den Benutzereingabewerten anzuzeigen.

13. Kit nach Anspruch 1, wobei der mindestens eine Tupfer (110) erste und zweite Tupfer (110a, 110b) umfasst, wobei der Hauptkörper (120) ein erster Hauptkörper ist, wobei der erste Tupfer lösbar in dem Innenraum des ersten Hauptkörpers installiert ist, und der Probennehmer ferner einen zweiten Hauptkörper mit einem Innenraum umfasst, wobei der zweite Tupfer lösbar in dem Innenraum des zweiten Hauptkörpers installiert ist und jeweils eine erste Einlassöffnung (121a, 121b) und eine erste Auslassöffnung (122) vorgesehen ist, um das Einführen bzw. Ausleiten von Außenluft zu ermöglichen; wobei die mindestens eine Trenneinheit eine erste Trenneinheit (200a) umfasst, die lösbar am ersten Hauptkörper (120a) installiert ist und dazu vorgesehen ist, das in einen ersten vorbestimmten Größenbereich fallende, in der durch die erste Einlassöffnung (121a) des ersten Hauptkörpers (121a) eingeführten Luft vorhandene Bioaerosol zuzuführen; eine zweite Trenneinheit (200b), die lösbar am zweiten Hauptkörper (120b) installiert ist und dazu vorgesehen ist, das in einen zweiten vorbestimmten Größenbereich fallende, in der durch die erste Einlassöffnung (121b) des zweiten Hauptkörpers (120b) eingeführten Luft vorhandene Bioaerosol zuzuführen, und wobei die Luftfördervorrichtung (300) jeweils mit den ersten Auslassöffnungen verbunden ist und dazu konfiguriert ist, die Außenluft in Richtung der ersten Auslassöffnungen durch die ersten Einlassöffnungen und die Innenräume, in denen die Tupfer installiert sind, strömen zu lassen, oder eine Luftfördervorrichtung jeweils mit den ersten Auslassöffnungen verbunden ist und dazu konfiguriert ist, den Luftstrom in den ersten Hauptkörper und den zweiten Hauptkörper zu führen.

## Revendications

1. Kit (10) de mesure d'un bioaérosol aéroporté et de matière particulaire, comprenant :
un échantillonneur (100 ; 100a ; 100b) comprenant au moins un écouvillon (110 ; 110a, 110b) prévu pour y attacher un bioaérosol aéroporté ; un corps principal (120) présentant un espace intérieur, dans lequel un écouvillon (110 ; 110a ; 110b) de l'au moins un écouvillon est installé de manière détachable dans l'espace intérieur, et présentant un premier orifice d'entrée (121) et un premier orifice de sortie (122) prévus, respectivement, pour permettre l'introduction et l'évacuation de l'air extérieur ; au moins une unité de séparation (200 ; 200a, 200b) installée de manière détachable sur le côté du premier orifice d'entrée (121) du corps principal (120) et prévue pour fournir le bioaérosol aéroporté, qui se trouve dans une plage de taille sélectionnée prédéterminée, dans l'air introduit à travers le premier orifice d'entrée (121) ; et un dispositif d'écoulement d'air (300) installé sur le côté du premier orifice de sortie (122) du corps principal (120) pour guider l'écoulement de l'air extérieur vers le premier orifice de sortie (122) à travers le premier orifice d'entrée (121) et l'espace intérieur dans lequel l'écouvillon (110 ; 110a, 110b) est installé en formant une pression différentielle de manière à permettre à l'air de s'écouler dans l'espace intérieur de l'au moins un corps principal (120) ; et
une unité de mesure d'adénosine triphosphate (ATP) (500) prévue pour mesurer l'ATP dans le bioaérosol aéroporté fixé à l'écouvillon (110 ; 110a, 110b) ;
**caractérisé en ce que**
le kit comprend en outre une unité d'humidification (400) prévue pour fournir une solution aqueuse contenant un agent de lyse de cellules vers l'air s'écoulant dans le corps principal (120) ;
une unité de mesure de matière particulaire (600) prévue pour mesurer une concentration de la matière particulaire, qui se situe dans une plage de taille prédéterminée, comprise dans l'air ; et
une unité de commande prévue pour recevoir des premières données délivrées en sortie de l'unité de mesure d'ATP (500) et des deuxièmes données délivrées en sortie de l'unité de mesure de matière particulaire (600) pour générer des troisièmes données sur la base d'au moins de données des premières et des deuxièmes données.

2. Kit (10) selon la revendication 1, dans lequel l'échantillonneur comprend en outre chacun de premiers et de deuxièmes trajets d'écoulement (251, 252) reliés en communication fluidique avec le premier orifice d'entrée (121) ; et une soupape (253) prévue sur le côté du premier orifice d'entrée (121) et prévue pour relier en communication fluidique le premier orifice d'entrée (121) au premier trajet d'écoulement (251) ou pour relier en communication fluidique le premier orifice d'entrée (121) au deuxième trajet d'écoulement (252),
dans lequel l'au moins une unité de séparation (200) comprend une première unité de séparation (200a) installée de manière détachable sur le côté du premier trajet d'écoulement (251) et prévue pour fournir le bioaérosol aéroporté, qui se situe dans une première plage de taille prédéterminée, dans l'air introduit par le premier orifice d'entrée (121) ; une deuxième unité de séparation (200b) installée de manière détachable sur le côté du deuxième trajet d'écoulement (252) et prévue pour fournir du bioaérosol aéroporté, qui se situe dans une deuxième plage de taille prédéterminée, dans l'air introduit par le premier orifice d'entrée (121).

3. Kit (10) selon la revendication 2, dans lequel le bioaérosol aéroporté situé une première plage de taille prédéterminée présente une plage de taille de 2,5 µm ou moins dans l'air, et le bioaérosol aéroporté situé dans la deuxième plage de taille prédéterminée présente une plage de taille de 10 µm ou moins dans l'air.

4. Kit (10) selon la revendication 1, dans lequel l'au moins une unité de séparation (200 ; 200a, 200b) comprend une première unité de séparation (200a) configurée pour fournir le bioaérosol aéroporté présentant une plage de taille de 2,5 µm ou moins dans l'air par le premier orifice d'entrée (121) et une deuxième unité de séparation (200b) configurée pour fournir le bioaérosol aéroporté présentant une plage de taille de 10 µm ou moins dans l'air par le premier orifice d'entrée (121), et au moins une des première et deuxième unités d'installation (200a, 200b) est installée.

5. Kit (10) selon la revendication 1, dans lequel l'unité de mesure de matière particulaire (600) est configurée pour mesurer chacune de la matière particulaire d'une taille de matière particulaire de 10 µm ou moins (PM10) et de la matière particulaire d'une taille de matière particulaire de 2,5 µm ou moins (PM 2,5) pour délivrer en sortie chacune des valeurs de mesure comme une valeur de concentration massique de la matière particulaire par unité de volume d'air.

6. Kit (10) selon la revendication 1, dans lequel l'unité de mesure d'ATP (500) comprend en outre un moniteur d'ATP (510) configuré pour délivrer en sortie chacune des valeurs d'ATP mesurées en tant que valeur d'unité de luminescence relative (RLU) par unité de volume d'air.

7. Kit (10) selon la revendication 1, comprenant en outre un terminal utilisateur configuré pour délivrer en sortie une interface utilisateur, dans lequel l'unité de commande est configurée pour fournir les troisièmes données générées au terminal utilisateur.

8. Kit (10) selon la revendication 7, dans lequel le terminal utilisateur est fourni pour entrer des valeurs d'entrée utilisateur, et les valeurs d'entrée utilisateur comprennent au moins un élément choisi parmi un débit volumique d'air d'échantillonnage, un temps d'échantillonnage, une formule de conversion d'unité formant colonie (UFC), une valeur de référence pour le bioaérosol aéroporté par masse ou volume de la matière particulaire d'une taille de matière particulaire de 10 µm ou moins pour chaque emplacement de mesure, et une valeur de référence pour le bioaérosol aéroporté par masse ou volume de la matière particulaire d'une taille de matière particulaire de 2,5 µm ou moins pour chaque emplacement de mesure.

9. Kit (10) selon la revendication 8, dans lequel l'unité de commande est configurée pour générer les troisièmes données sur la base des valeurs d'entrée utilisateur et d'au moins des données des premières données et des deuxièmes données.

10. Kit (10) selon la revendication 9, dans lequel les troisièmes données comprennent au moins une donnée choisie parmi une valeur RLU du bioaérosol aéroporté présentant une plage de taille de 10 µm ou moins dans l'air par unité de masse de la matière particulaire (RLU/µg [PM 10]), une valeur RLU du bioaérosol aéroporté présentant une plage de taille de 2,5 µm ou moins dans l'air par unité de masse de la matière particulaire (RLU/µg [PM 2,5]), une valeur RLU du bioaérosol aéroporté présentant une plage de taille de 2,5 µm à 10 µm dans l'air par unité de masse de la matière particulaire (RLU/µg [PM 10-PM 2,5]), une valeur d'unité formant colonie (UFC) du bioaérosol aéroporté présentant une plage de taille de 10 µm ou moins par unité de masse de la matière particulaire (UFC/µg [PM 10]), une valeur d'unité formant colonie (UFC) du bioaérosol aéroporté présentant une plage de taille de 2,5 µm ou moins dans l'air par unité de masse de la matière particulaire (UFC/µg [PM 2,5]), une valeur d'unité formant colonie (UFC) du bioaérosol aéroporté présentant une plage de taille de 2,5 µm à 10 µm dans l'air par unité de masse de la matière particulaire (UFC/µg [PM 10-PM 2,5]), une valeur d'unité formant colonie (UFC) du bioaérosol aéroporté présentant une plage de taille de 10 µm ou moins dans l'air par unité de volume d'air (UFC/m³ [PM 10]), une valeur d'unité formant colonie (UFC) du bioaérosol aéroporté présentant une plage de taille de 2,5 µm ou moins dans l'air par unité de volume d'air (UFC/m³ [PM 2,5]), une valeur d'unité formant colonie (UFC) du bioaérosol aéroporté présentant une plage de taille de 2,5 µm à 10 µm dans l'air par unité de volume d'air (UFC/m³ [PM 10-PM 2,5]), et une valeur de concentration massique (µg/m³) de la matière particulaire présentant une taille de particule supérieure à 2,5 µm et moins à 10 µm par unité de volume d'air.

11. Kit (10) selon la revendication 7, dans lequel l'interface utilisateur comprend en outre une fenêtre d'entrée utilisateur (1000) configurée pour entrer au moins une valeur d'entrée utilisateur, une troisième fenêtre de sortie de données (2000) configurée pour délivrer en sortie les troisièmes données générées, et une troisième fenêtre de sortie de schématisation de données (3000) configurée pour fournir chacun des éléments des troisièmes données sous une forme schématisée.

12. Kit (10) selon la revendication 11, dans lequel la troisième fenêtre de sortie de schématisation de données (3000) est configurée pour afficher, sous la forme d'une ligne droite horizontale (v1, v2, v3, v4), chacune des lignes de référence représentant une valeur de référence pour le bioaérosol aéroporté et/ou des valeurs de référence pour la matière particulaire d'une taille de 10 µm ou moins et la matière particulaire d'une taille de 2,5 µm ou moins pour chaque emplacement de mesure sur la base des valeurs d'entrée utilisateur.

13. Kit selon la revendication 1, dans lequel l'au moins un écouvillon (110) comprend des premiers et deuxièmes écouvillons (110a, 110b), dans lequel le corps principal (120) est un premier corps principal, dans lequel le premier écouvillon est installé de manière détachable dans l'espace intérieur du premier corps principal et l'échantillonneur comprend en outre un deuxième corps principal présentant un espace intérieur,
dans lequel le deuxième écouvillon est installé de manière détachable dans l'espace intérieur du deuxième corps principal,
et présentant un premier orifice d'entrée (121a, 121b) et un premier orifice de sortie (122) prévus respectivement pour permettre l'introduction et l'évacuation de l'air extérieur ;
dans lequel l'au moins une unité de séparation comprend une première unité de séparation (200a) installée de manière détachable sur le premier corps principal (120a) et prévue pour fournir le bioaérosol aéroporté, qui se situe dans une première plage de taille prédéterminée, dans l'air introduit par le premier orifice d'entrée (121a) du premier corps principal (121a) ; une deuxième unité de séparation (200b) installée de manière détachable sur le deuxième corps principal (120b) et prévue pour fournir le bioaérosol aéroporté, qui se situe dans une deuxième plage de taille prédéterminée, dans l'air introduit par le premier orifice d'entrée (121b) du deuxième corps principal (120b), et dans lequel le dispositif d'écoulement d'air (300) est relié à chacun des premiers orifices de sortie et est configuré pour guider l'écoulement de l'air extérieur vers les premiers orifices de sortie par les premiers orifices d'entrée et les espaces intérieurs, dans lesquels les échantillons sont installés ou un dispositif d'écoulement d'air est relié à chacun des premiers orifices de sortie et est configuré pour guider l'écoulement d'air dans le premier corps principal et le deuxième corps principal.
